# EUROPÄISCHE PATENTANMELDUNG

(11) **EP 1 878 423 A2**
(43) Veröffentlichungstag der Anmeldung: **16.01.2008**
(21) Anmeldenummer: 07110950.8
(22) Anmeldetag: 25.06.2007
(51) Int. Cl.: A61K 8/64, A61Q 5/06

(54) **Haarstylingzubereitungen mit besonderen Proteinhydrolysaten**

(30) Priorität: 13.07.2006 DE 102006032665
(71) Anmelder: Beiersdorf AG, 20253 Hamburg (DE)
(72) Erfinder: Detert, Marion, 22455, Hamburg (DE); Dingler, Christian, 22527, Hamburg (DE); Pilzner, Anke, 22459 Hamburg (DE); Sass, Viola, 25488, Holm (DE)

(57) **Zusammenfassung**

Kosmetische Zubereitung enthaltend
a) Proteinhydrolysate aus Kaschmir-Wolle,
b) mindestens ein fixierendes Polymer.

## Beschreibung

Die Erfindung betrifft kosmetische Zubereitungen, insbesondere Haarstylingzubereitung, enthaltend Proteinhydrolysate aus Kaschmir-Wolle und fixierende Polymere. Die Zubereitungen sind mild zu Haut und Haaren und führen zu einer Verbesserung der Haarstruktur als auch der physikalisch-optischen Haareigenschaften.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei das Aussehen der Haare. Das Haar wird gerne frisiert, also in Form gebracht. Diese gestaltende Tätigkeit wird auch als "Stylen" bezeichnet.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem so genannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur temporären Verformung des Haares und Gestaltung (engl. Styling) vielseitiger Frisuren verwendet man Haarfestiger, meist in Form von Schaumfestigern oder Haarsprays. Beide haarkosmetischen Mittel sind in Ihrer Zusammensetzung ähnlich, unterscheiden sich aber in ihrer Aufgabe und Anwendung. Schaumfestiger (auch Schaumhaarfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele angeboten.

Die Rezepturen für derartige Produkte sind vielfältig. Die Bestandteile müssen jedoch in ethanolischem, wässrig-ethanolischem oder wässrigem Medium verträglich sein.

Üblicherweise bestehen diese Mittel zur Festigung der Haare (Haarfestiger) aus Lösungen von filmbildenden natürlichen oder synthetischen Polymeren oder Polymerkombinationen. Es handelt sich dabei um nichtionische, anionische, kationische oder amphotere Polymere. Diese hinterlassen auf dem Haar nach der Applikation meist einen transparenten farblosen Film. Es werden eine Reihe von Anforderungen an diese Filme gestellt: sie sollen klar, glänzend, feuchtigkeitsunempfindlich, festigend, lang anhaltend, flexibel, nicht klebend sein, den natürlichen Griff des Haares nicht beeinträchtigen und sich auch wieder leicht durch handelsübliche Haarwaschmittel auswaschen lassen. Schaumfestiger besitzen in der Gruppe der Stylingprodukte ein Sonderstellung. Nicht nur die Filmeigenschaften (Halt, Flexibilität...) stehen im Vordergrund, sondern auch die Pflegeleistung am Haar (Kämmbarkeit, gepflegter Griff) und die Schaumcharakteristik (feinblasig, cremig, stabil) haben einen beträchtlichen Einfluss auf die Produktperformance.

Die Auswahl des geeigneten Polymers (nichtionisch, anionisch, kationisch oder amphoter) ist von entscheidender Bedeutung für die Eigenschaften des Endproduktes.

Kationische Filmbildner weisen vor allem pflegende Eigenschaften auf: das Haar lässt sich gut kämmen und fühlt sich geschmeidig und gepflegt an. Insbesondere für Schaumfestiger hat die Kämmbarkeit und der Griff der nassen und trockenen Haare eine entscheidende Bedeutung. Als entscheidender Nachteil dieser Rohstoffe ist ihre geringe Festigungsleistung zu nennen.

Anionische oder amphotere Filmbildner werden in Haarfestigern eingesetzt, um eine gute und lang anhaltende Festigung der Frisur zu erreichen. Ein entscheidender Nachteil dieser Rohstoffe sind jedoch ihre mangelhaften pflegenden Eigenschaften: das Haar lässt sich schlecht kämmen und fühlt sich stumpf, rau und wenig gepflegt an. Nach dem Trocknen sind die Filme dann relativ hart, spröde und unflexibel. Nachteilig für den Verbraucher sind die taktilen Eigenschaften des Filmes sowie die Bildung von Rückständen beim Kämmen (Filmplaken). Diese rieseln auf Schultern oder Kleidung und lassen den Anwender ungepflegt erscheinen. Bei Schaumfestigern kommt darüber hinaus ein weiterer Nachteil vieler anionischer und amphoterer Polymere zum Tragen: sie verleihen dem Schaum eine unzureichende, unkosmetische Schaumcharakteristik. Der Schaum ist in der Regel grobblasig, weich, instabil und wenig cremig kann aber auch sehr fest und cremig sein.

Bei Produkten zum Stylen der Haare die anionische oder amphotere Polymere enthalten , insbesondere Aerosole besteht ein seit langem bekanntes Problem darin, dass der Polymerfilm, der die Haare nach der Anwendung des Produktes fixiert meist spröde ist und beim Auskämmen kosmetisch unschöne, sichtbare Partikel, die vom Verbraucher oft als "Schuppen" wahrgenommen werden, hinterlässt.

Es war daher die Aufgabe der vorliegenden Erfindung, die Mängel des Standes der Technik zu beseitigen.

Die herkömmlichen Proteinhydrolysate haben eine stark ausgeprägte Neigung zu Verfärbungen sowohl bei Einarbeitung in eine Formulierung als auch bei anschließenden Lagerung. Des Weiteren kommt es hier besonders häufig zu negativen geruchlichen Veränderungen des Rohstoffes, der auch die Gesamtformulierung negativ beeinflussen kann. Hierdurch müssen bei einem Einsatz dieser Proteinhydrolysate die fixierende Polymere enthaltenden Formulierungen mit entsprechenden Zusatzstoffen wie Stabilisatoren oder einer erhöhten Parfümkonzentration versehen werden.

Gelöst wird dieses Bündel an Aufgaben durch eine kosmetische Zubereitung enthaltend Proteinhydrolysate aus Kaschmir-Wolle und mindestens ein fixierendes Polymer. Das Haar fühlt sich nach der Anwendung nicht mehr stumpf an, sondern hinterlässt einen glatten, gut gepflegten Eindruck. Die Bildung von Filmplaken wird unterdrückt. Festigungsleistung und Langzeithalt sind gegenüber dem Stand der Technik erhöht. Dabei ist es bevorzugt, wenn das Proteinhydrolysat aus Kaschmirwolle ein Gewichtsmittel des Molekulargewichtes von 5000 bis 8000, besonders bevorzugt 6500 bis 7500 hat. Weiter ist es bevorzugt, wenn der Gehalt an Proteinhydrolysat 0,001 bis 5 Gew.-% beträgt, besonders bevorzugt 0,01 bis 2 Gew.-%, ganz besonders bevorzugt 0,05 bis 1,5 Gew.-%. Dabei ist es bevorzugt, wenn als fixierendes Polymer bei Haarsprays ein amphoteres Polymer, bei Schaumfestigern ein nichtionisches Polymer und bei Frisiergelen ein nichtionisches und/oder anionisches Polymer enthalten ist. Dabei ist es erfindungsgemäß vorteilhaft, wenn die erfindungsgemäße Zubereitung 0,01 bis 50 Gew.-% und bevorzugt 8 bis 11 Gew.-% bei Schaumfestigern bzw. 35 - 50 Gew.-% bei Haarsprays, jeweils bezogen auf das Gesamtgewicht der Zubereitung, Treibmittel enthält. Erfindungsgemäß vorteilhafte Treibmittel sind Dimethylether, Propan, Isobutan und n-Butan sowie deren Mischungen. Erfindungsgemäß bevorzugt wird als Treibmittel bei Schaumfestigern eine Mischung aus Propan, Isobutan und Butan eingesetzt, bei Haarsprays Dimethylether. Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Weiter ist es bevorzugt, wenn in der erfindungsgemäßen Zubereitung wenigstens 5 Gew.-% Wasser enthalten ist. Weiter ist es bevorzugt, wenn das anionische Polymer aus der Gruppe Vinylacetat/ Crotonsäure Copolymere,
Terpolymere aus Vinylacetat, Crotonat und Vinylalkanoat,
partialveresterte Copolymere zwischen Vinylmethylether und Malein-säureanhydrid, Copolymere aus Acrylsäure oder Methacrylsäure mit Alkylacrylaten und/oder N-Alkylacrylamiden,
Terpolymere aus Acryl-säure, Alkylacrylaten und N-Alkylacrylamiden,
tert.-Butylacrylat/Ethylacrylat/ Methacrylsäure Terpolymer,
Polystyrolsulfonate,
in Wasser lösliche oder dispergierbare anionische Polyurethane
gewählt wird. Geeignet sind Vinylacetat/ Crotonsäure Copolymere (INCI VA/Crotonates Copolymer) z. B. Resyn 28-1310 von National Starch oder Luviset CA66 von BASF; Terpolymere aus Vinylacetat, Crotonat und Vinylalkanoat, insbesondere Vinylacetat/Crotonat/Vinylnoedecanoat Copolymere (INCI: VA/Crotonates/Vinylneodecanoate Copolymer) z. B. Resyn 28-2930 von National Starch; partialveresterte Copolymere zwischen Vinylmethylether und Malein-säureanhydrid (INCI: Ethyl-, Isopropyl-,Butylester of PVM/MA Copolymer) z. B. Gantrez ES 225 oder Gantrez ES 425 von ISP; Copolymere aus Acrylsäure oder Methacrylsäure mit Alkylacrylaten und/oder N-Alkylacrylamiden, insbesondere Co-polymere aus Methacrylsäure und Alkyacrylaten sowie Terpolymeren aus Acryl-säure, Alkylacrylaten und N-Alkylacrylamiden wie Acrylsäure/Ethylacrylat/N-tert.-Butylacrylamid Terpolymer (INCI: Acrylate/Acrylamide Copolymer) z. B. Ultrahold 8 von BASF oder tert.-Butylacrylat/Ethylacrylat/ Methacrylsäure Terpolymer (INCI: Acrylates Copolymer (6)), z. B. Luvimer von BASF; Polystyrolsulfonate (INCI: Sodium Polystyrene Sulfonate) z. B. Flexan 130 von National Starch.

Geeignete anionische Polymere sind auch in Wasser lösliche oder dispergierbare anionische Polyurethane, z. B. Luviset PUR von BASF oder Polyester.

Besonders bevorzugt ist Acrylates Copolymer (6) oder Sodium Polystyrene Sulfonate.

Weiter ist es bevorzugt, wenn das amphotere Polymer aus der Gruppe Octylacrylamide/Acrylates/Butylaminoethyl Methacrylate Copolymer, Acrylates/Lauryl Acrylate/Stearyl Acrylate/Ethylamine Oxide Methacrylate Copolymer, Methacryloyl Ethylbetaine/MethAcrylates Copolymer (6), Copolymerisate aus Carboxylgruppen oder Sulfongruppen enthaltenden Monomeren, Copolymere aus N-Octylacrylamid, Methylmethacrylat, Hydroxypropylmethacrylat, N-tert.-Butylaminoethylmethacrylat und Acrylsäure
gewählt wird. Geeignet sind
Octylacrylamide/Acrylates/Butylaminoethyl Methacrylate Copolymer (Amphomer 28-4910; National Starch), Acrylates/Lauryl Acrylate/Stearyl Acrylate/Ethylamine Oxide Methacrylate Copolymer (Diaformer; Mitsubishi) oder Methacryloyl Ethylbetaine/MethAcrylates Copolymer (6) (Diaformer; Mitsubishi). Weiterhin sind geeignet Copolymerisate aus Carboxylgruppen oder Sulfongruppen enthaltenden Monomeren, z. B. (Meth)Acrylsäure und Itaconsäure mit basischen insbesondere Aminogruppen enthaltenden Monomeren wie z. B. Mono- bzw. Dialkylaminoalkyl(meth)acrylaten und/oder Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure.

Besonders bevorzugt ist hierbei Octylacrylamide/Acrylates/Butylaminoethyl Methacrylate Copolymer.

### Weiter bevorzugt ist es, wenn das nichtionische Polymer aus der Gruppe

VP/VA Copolymer, PVP, Polyvinylalkohol, Polyvinylcaprolactam, VP/Vinylcaprolactam/Vinyl Imidazole, VP/ Methacrylamide/N-Vinyl Imidazole Copolymer, PVP/Dimethylaminoethylmethacrylate Copolymer gewählt wird.

Bei allen Produktformen ist es weiter bevorzugt, wenn ein zusätzliches kationisches Polymer enthalten ist, besonders bevorzugt aus der Gruppe Polyquaternium-4, Polyquaternium-68, Polyquaternium-16, Polyquaternium-11, Polyquaternium-46 gewählt.

Besonders bevorzugt ist es, wenn die Zubereitung Crotein Kaschmir oder Cashmilan LS 9604 enthält. Die Erfindung umfasst auch die Verwendung einer solchen Zubereitung zur Gestaltung der Haarfrisur, sowie die Verwendung einer solchen Zubereitung zur stabilen Bereitstellung von erfindungsgemäßen Proteinhydrolysaten in Haarkosmetikprodukten.

Die Erfindung umfasst darüber hinaus auch die Verwendung der Zubereitungen auf der Haut und/oder dem Haar. Für den Fachmann überraschend wurde festegestellt, dass Extrakte von Ziegenhaar, hier insbesondere die der Kaschmirziege, bisher in kosmetischen Formulierungen, hier insbesondere tensidhaltige Formulierungen, noch nicht zum Einsatz gekommen sind.

Für Haarreinigungsformulierungen hat sich eine verbesserte Kämmbarkeit gezeigt. Auch hier ist ein positiver Einfluss auf die Sensorik zu spüren, der sich in einem angenehmeren Griff äußert.

Aufgrund des natürlichen Ursprungs dieses Rohstoffes zeigt dieser eine gute biologische Abbaubarkeit und eine gute Hautverträglichkeit.

Die Erfindung umfasst auch die Verwendung von erfindungsgemäßen Proteinhydrolysaten zur Verbesserung der Eigenschaften der fixierenden Polymere in kosmetischen Haarstylingzubereitungen. Diese Verbesserung besteht im Verhindern des "Flaking Effekts", der Verringerung der Rückstände beim Auskämmen, einem besseren Griff der Haare, einer Plastifizierung des Polymerfilms auf dem Haar.

Die Aufgabe umfasst weiterhin die stabile Einarbeitung dieses oxidationsempfindlichen Gemisches. Extrakte aus Haaren der Kaschmirziege lassen sich überraschenderweise besonders gut und stabil in polymerhaltige Produkte einarbeiten, ohne dass ein weiterer Einsatz von so genannten Hilfsstoffen zur Stabilisierung unbedingt erforderlich ist.

Optionale herkömmliche Additive können ebenfalls in die Formulierung einbezogen sein, um der Zusammensetzung bestimmte Modifizierungseigenschaften zu verleihen: dies sind Silikone oder Silikonderivate, Lösungsmittel, Benetzungsmittel, Feuchthaltemittel, Weichmacher wie Glycerin, Glykol und Phthalester und - ether, erweichende Mittel, Riechstoffe und Parfüms, UV, Absorber, Farbstoffe , Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer, Verdicker und Rheologiemodifizierer, Geliermittel, Trübungsmittel, Stabilisatoren, Sequestierungsmittel, Komplexbildner, Perlglanzmittel, ästhetische Verstärker, Fettsäuren, Fettalkohole, Triglyceride, botanische Extrakte, Klärhilfsmittel und Filmbildner.

Diese Additive sind im allgemeinen in einer Konzentration von etwa 0,01 % bis 10 Gew.-% bezogen auf das Gesamtgewicht der Zusammensetzung vorhanden.

Die aufgeführte Liste an Zusatzstoffen soll selbstverständlich nicht limitierend sein.

Erfindungsgemäß bevorzugt ist die Verwendung der kosmetische Zubereitung als festigendes Mittel für die Haare. Erfindungsgemäß besonders bevorzugt ist die Verwendung der erfindungsgemäßen Zubereitung als Schaumfestiger oder Haarspray für die Frisurgestaltung.

Proteinhydrolisate der Kaschmir-Wolle sind bereits beschrieben. Sie werden beispielsweise von Laboratoires Serobiologiques, Frankreich, hergestellt und vertrieben und als "Cashmilan LS 9604" vertrieben..

Ein zusätzlicher Gehalt an Antioxidantien ist in kosmetischen und/oder dermatologischen Zubereitungen im allgemeinen bevorzugt.

Die Mittel gemäß der Erfindung können beispielsweise aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels.

Als Treibmittel für aus Aerosolbehältern versprühbare kosmetische oder dermatologische Zubereitungen im Sinne der vorliegenden Erfindung, sind die üblichen bekannten leichtflüchtigen, verflüssigten Treibmittel, beispielsweise Dimethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Es kann auch von Vorteil sein, erfindungsgemäße Zubereitungen mit UV-A-Filtern zu versetzen, die bisher üblicherweise in kosmetischen Zubereitungen enthalten sind. Es können die für die UV-B-Kombination verwendeten Mengen eingesetzt werden.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Der pH-Wert der Zubereitungen kann in bekannter Weise durch Zugabe von Säuren oder Basen eingestellt werden, vorzugsweise durch Zugabe von Puffergemischen, z. B. auf Basis von Citronensäure/Citrat oder Phosphorsäure Phosphat-Puffergemischen. Vorzugsweise liegt der pH-Wert unter 10, z. B. bei Schaumfestigern und Gelen im Bereich von 2 - 7, insbesondere im Bereich von 3 - 5 und bei Haarsprays (auch Haarfestiger genannt) im Bereich von 7 - 10.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

### Beispielrezepturen Haarfestiger + Kaschmir

### Beispiele 1 - 12

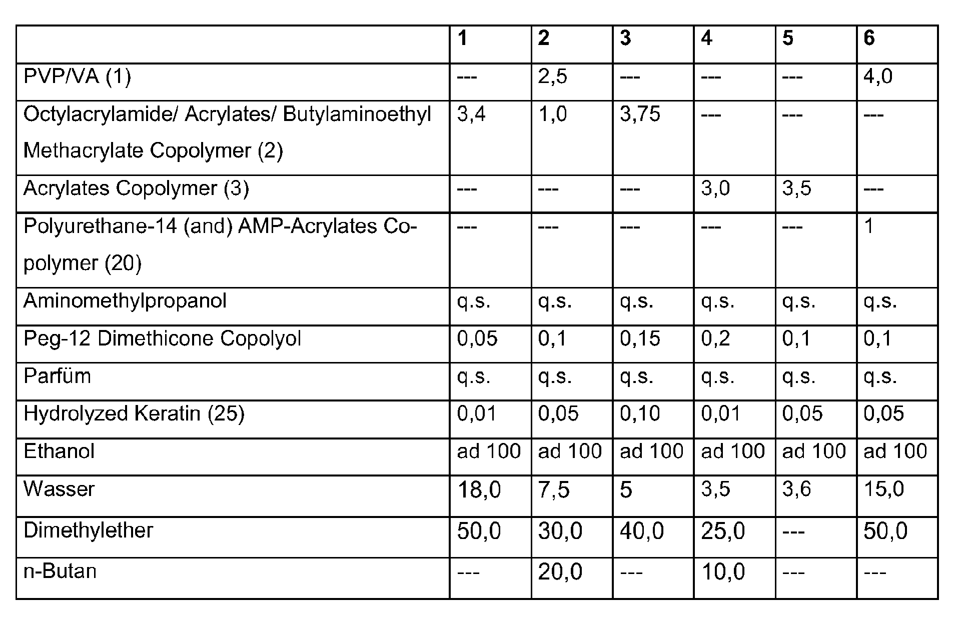

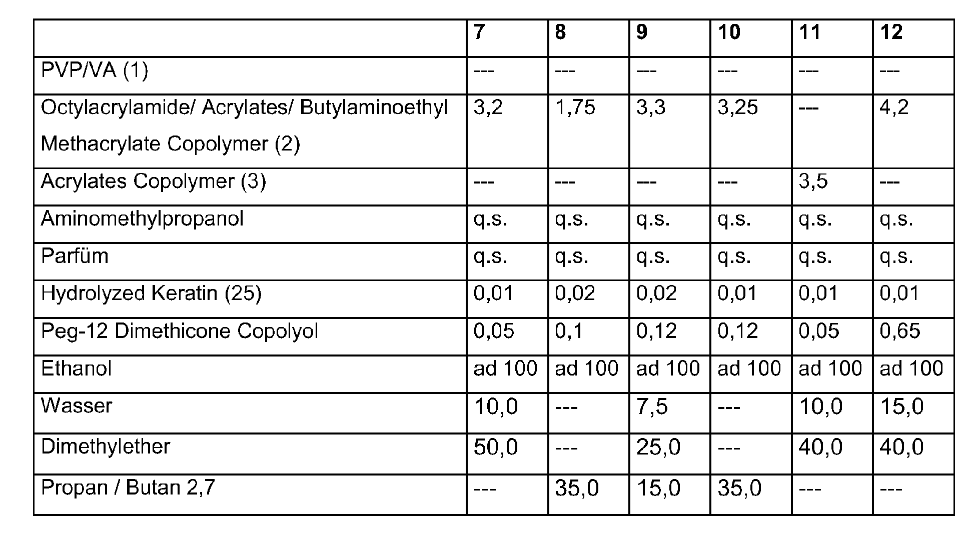

### Beispielrezepturen Emulsionen + Kaschmir

### Rezepturbeispiele:

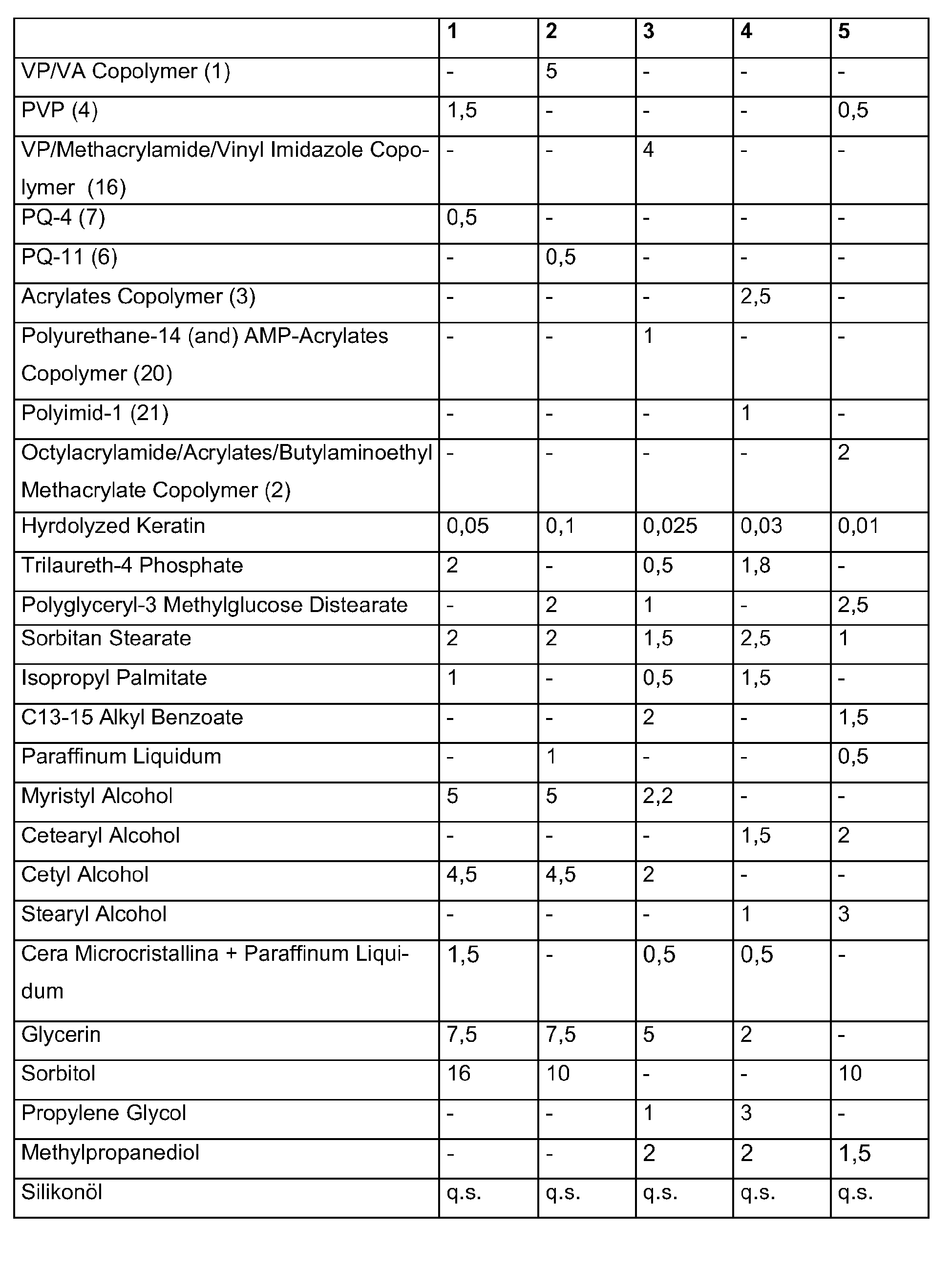

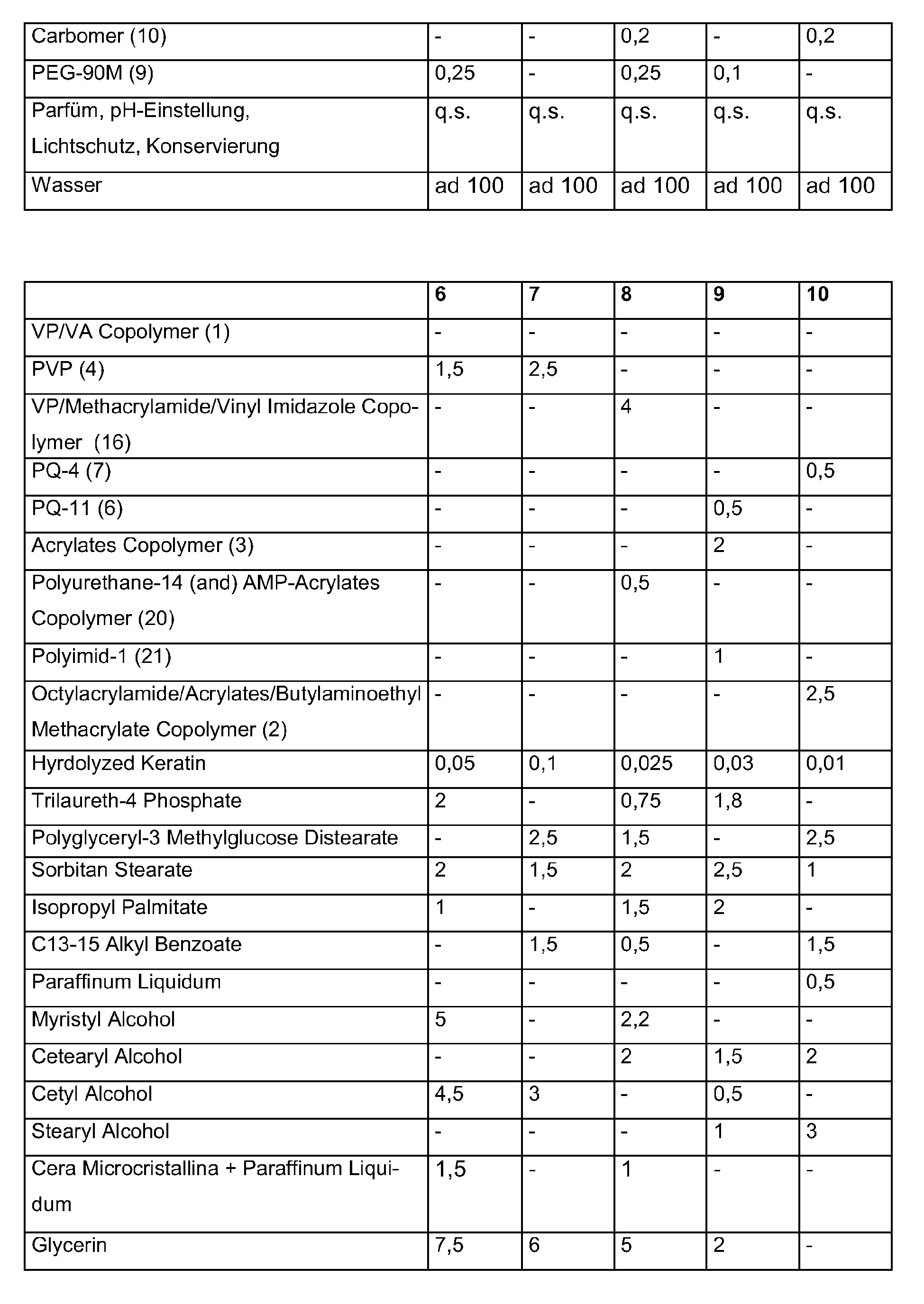

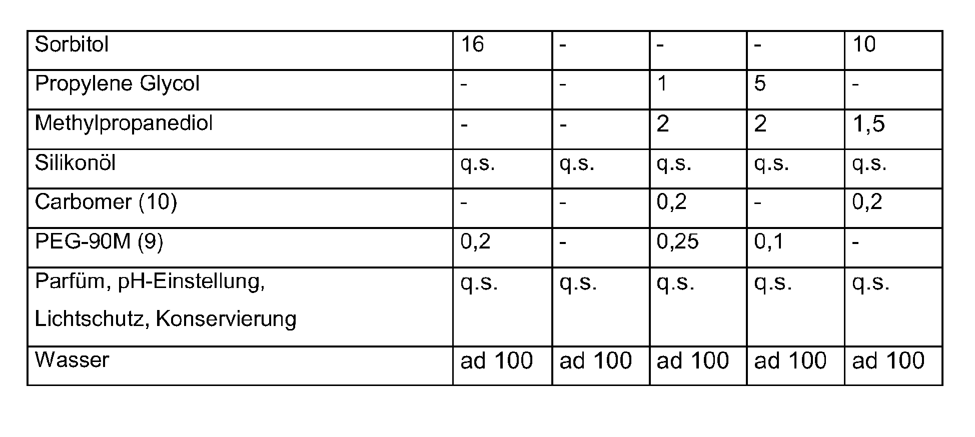

### Beispielrezepturen Hydrodispersion + Kaschmir

### Rezepturbeispiele :

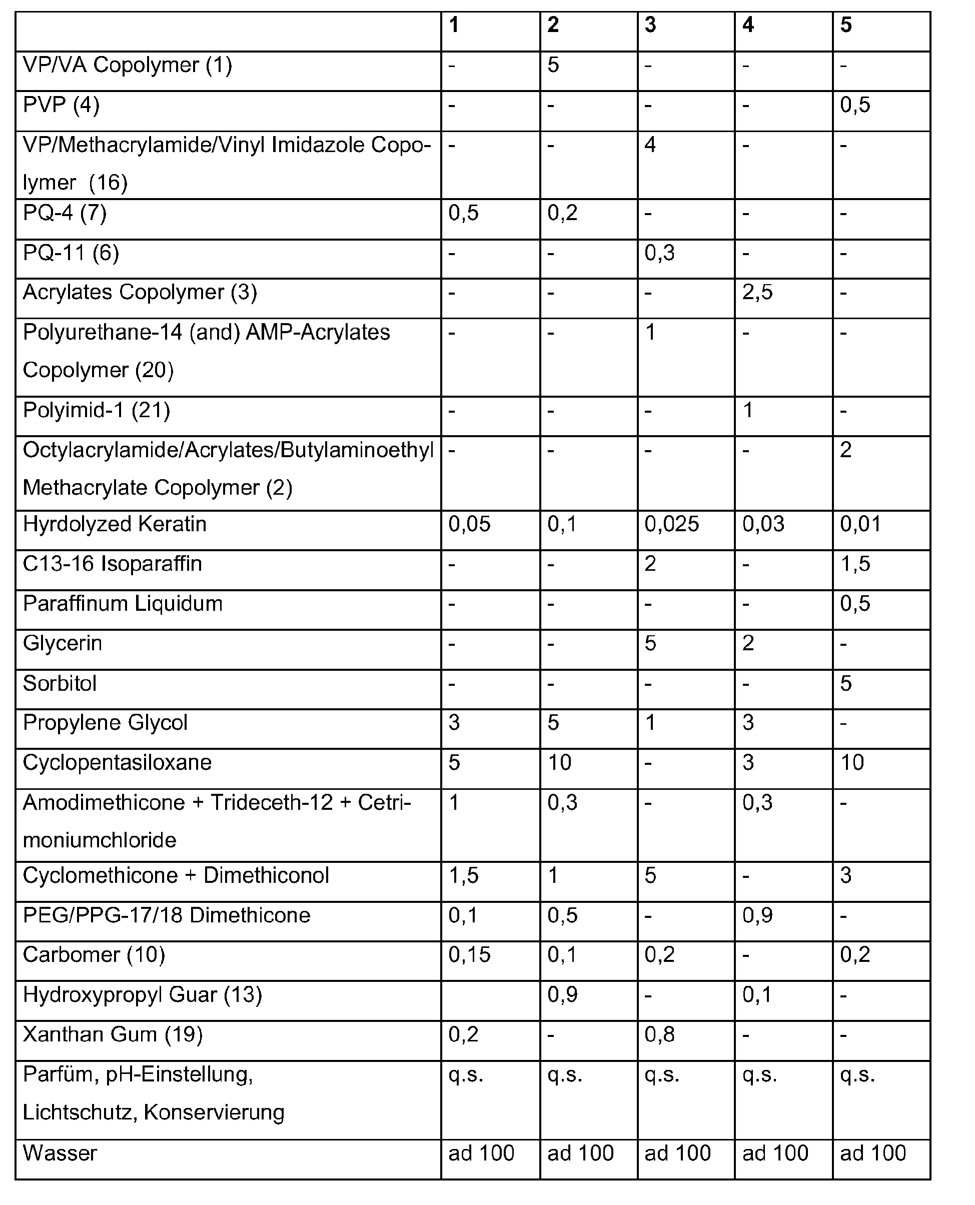

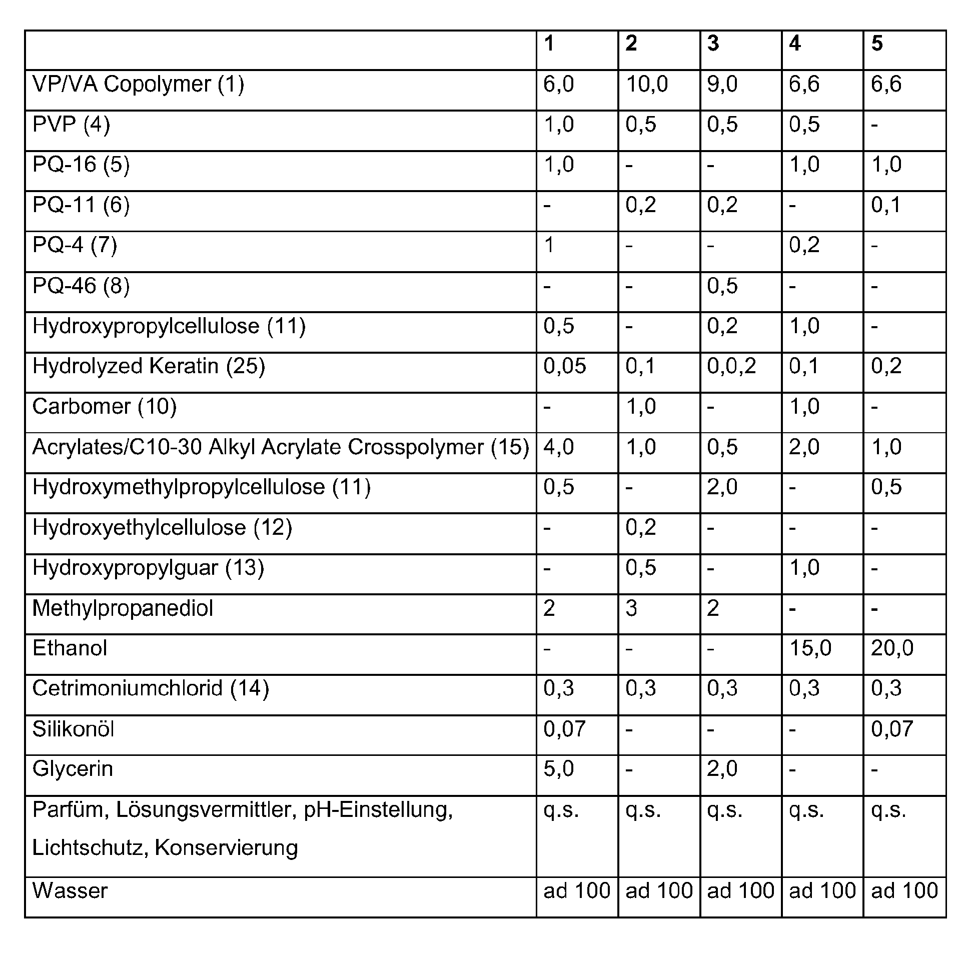

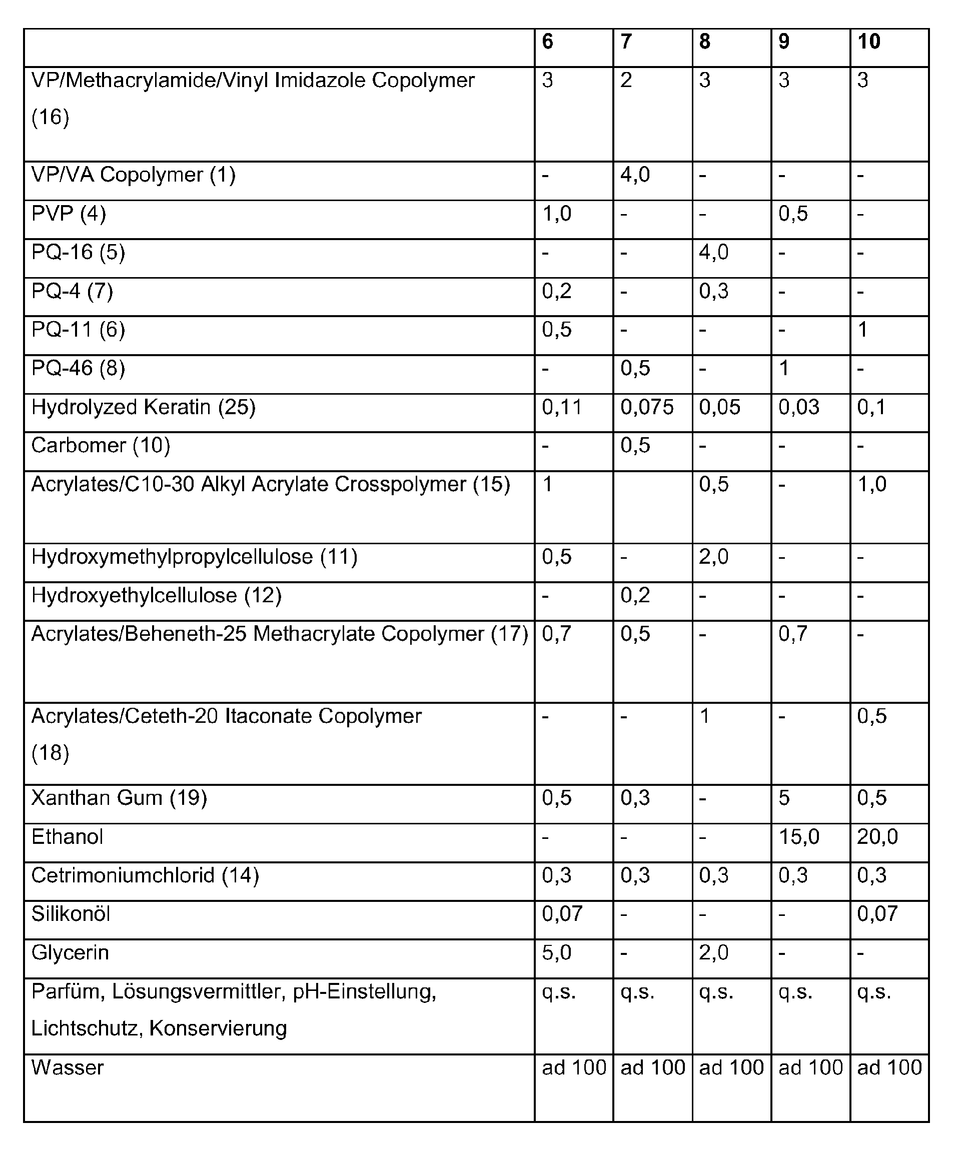

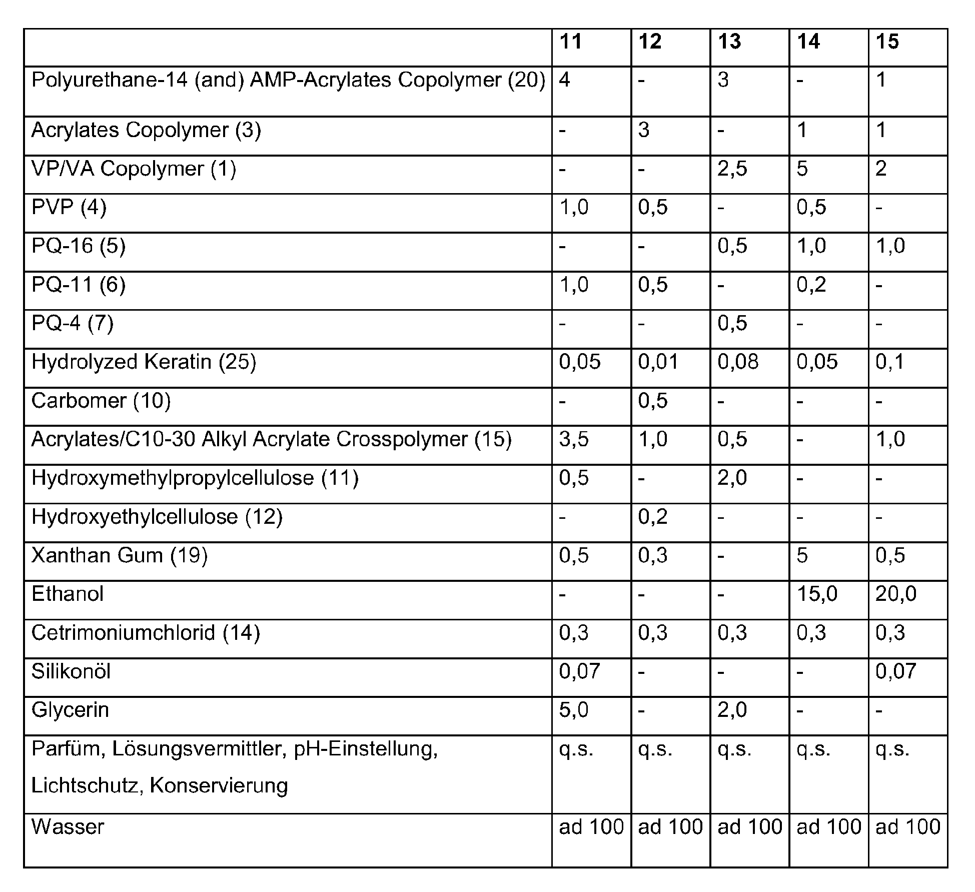

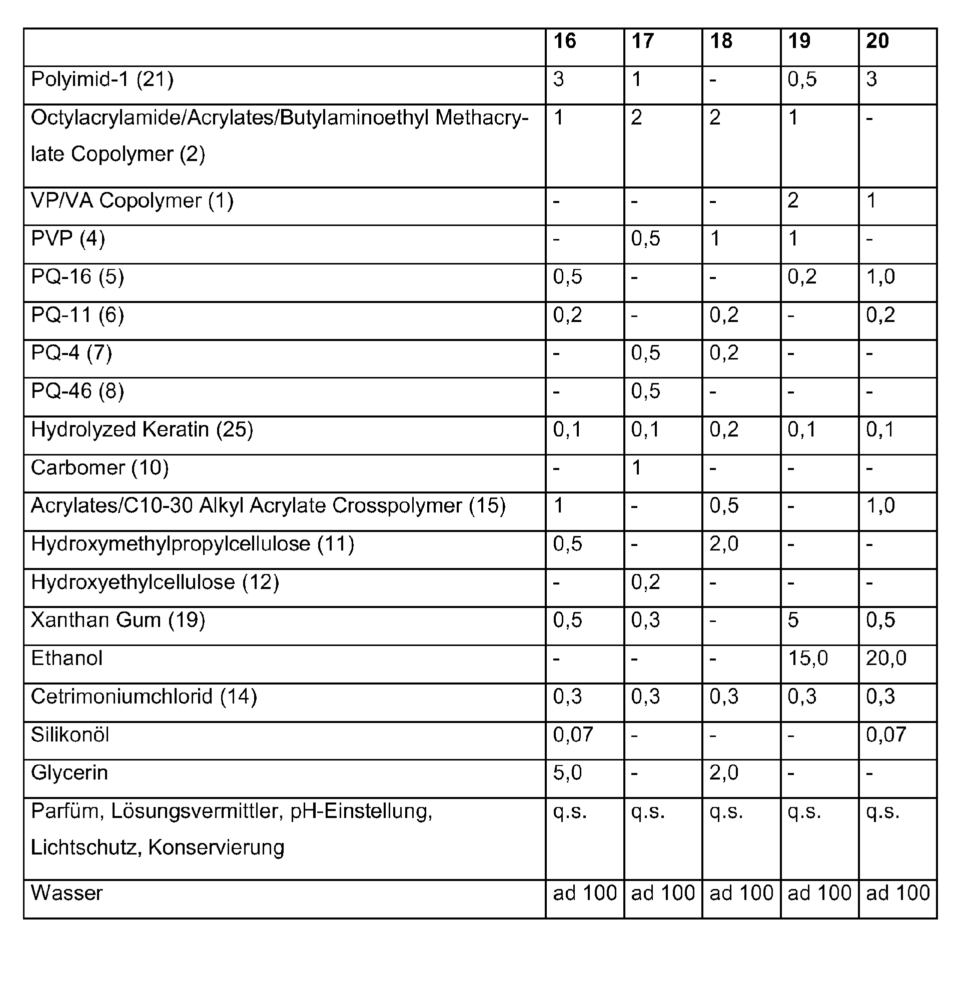

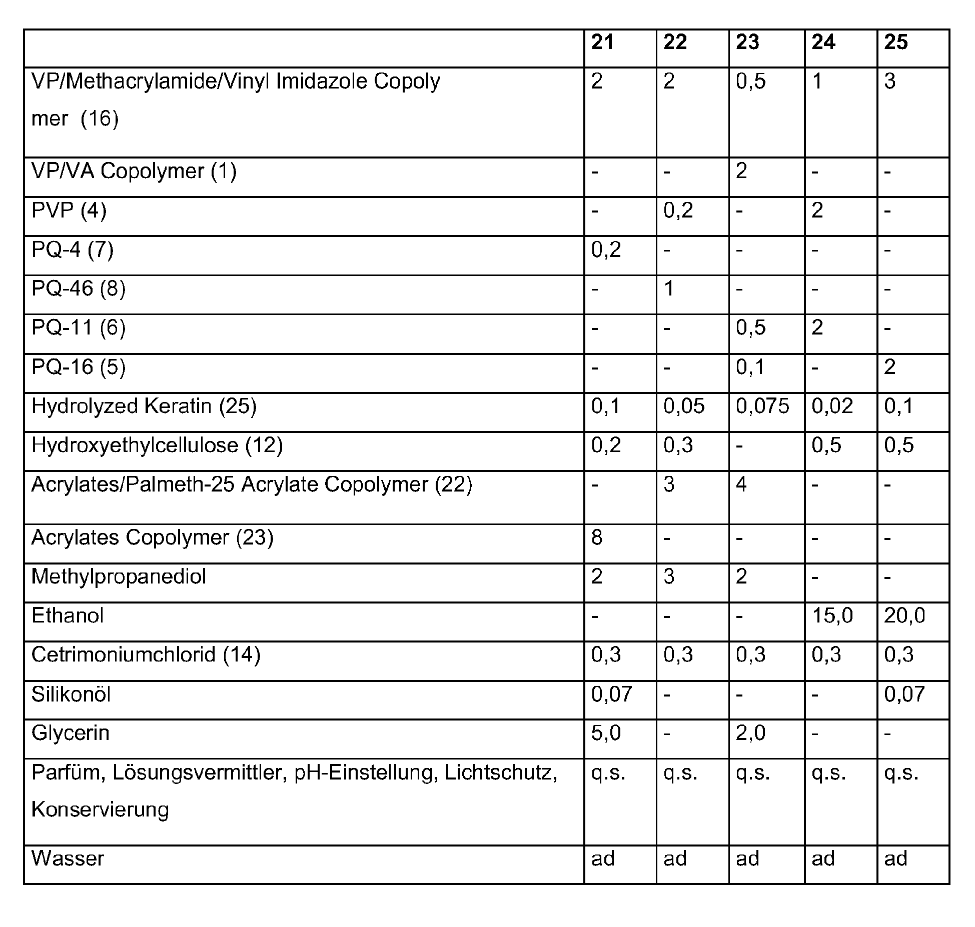

### Beispielrezepturen Schaumfestiger + Kaschmir

| | **1** | **2** | **3** | **4** | **5** | **6** |
|---|---|---|---|---|---|---|
| VP/VA Copolymer (1) | 4,0 | - | - | | 1,0 | - |
| PVP (4) | | 2,0 | - | 2,5 | - | 0,5 |
| Polyquaternium-68 (24) | 1,0 | 0,2 | - | 0,8 | - | 0,5 |
| Polyquaternium -4 (7) | 0,15 | - | - | - | - | - |
| Polyurethane-14 (and) AMP-Acrylates Co-polymer (20) | - | - | - | - | 3 | - |
| Polyimid-1 (21) | - | - | - | - | - | 3 |
| Cetrimoniumchloride (14) | 0,3 | 0,2 | 0,3 | 0,3 | 0,2 | 0,3 |
| Hydroxyethyl Cetyldimonium Phosphate | 0,5 | - | - | 0,4 | - | 0,2 |
| Panthenol | 0,1 | 0,1 | 0,1 | 0,1 | 0,1 | 0,1 |
| Niacinamide | 0,02 | 0,02 | - | 0,02 | - | - |
| Hydrolyzed Keratin (25) | 0,1 | 0,05 | 0,025 | 0,1 | 0,05 | 0,025 |
| Hydroxypropyl Methylcellulose (11) | 0,25 | - | - | 0,1 | - | - |
| Parfüm, Lösungsvermittler, pH-Einstellung, Konservierung | q.s. | q.s. | q.s. | q.s. | q.s. | q.s. |
| Isobutan/Propan/Butan | 10 | 10 | 10 | 10 | 10 | 10 |
| Ethanol | - | 10 | 10 | - | 10 | - |
| Wasser | ad 100 | ad 100 | ad 100 | ad 100 | ad 100 | ad 100 |

### Stoffliste:

(1) Luviskol Va 64 W von BASF
(2) Amphomer 28-4910 von National Starch
(3) Luvimer 100P von BASF
(4) Luviskol K-Typen von BASF
(5) Luviquat FC 370 von BASF
(6) Gafquat 755N von ISP
(7) Celquat L200 von National Starch
(8) Luviquat Hold von BASF
(9) Polyox-Typen von Dow Chemical z.B. Polyox WSR-301
(10) Carbopol 980 oder Carbopol Ultrez 10 von Noveon
(11) Klucel EF oder HF von Hercules
(12) Natrosol 250 von Hercules
(13) Jaguar-Typen von Rhodia
(14) Dehyquart A-CA von Cognis oder CTAC 6025 von KCI
(15) Carbopol Ultrez 21 oder Carbopol ETD 2020 oder Carbopol 1382 von Noveon
(16) Luviset Clear von BASF
(17) Aculyn 28 von Rohm&Haas
(18) Structure 3001 von National Starch
(19) Keltrol F von Kelco
(20) DynamX von National Starch
(21) Aquaflex-30 von ISP
(22) Synthalen W 2000 von 3V Sigma
(23) Carbopol Aqua SF-1 von Noveon
(24) Luviquat Supreme von BASF
(25) Cashmilan LS 9604 Laboratoires Serobiologiques S.A.

## Patentansprüche

1. Kosmetische Zubereitung enthaltend
a) Proteinhydrolysate aus Kaschmir-Wolle,
b) mindestens ein fixierendes Polymer.

2. Zubereitung nach Anspruch 1 wobei das Proteinhydrolysat aus Kaschmirwolle ein Gewichtsmittel des Molekulargewichtes von 5000 bis 8000, besonders bevorzugt 6500 bis 7500 hat.

3. Zubereitung nach Anspruch 1 wobei der Gehalt an Proteinhydrolysat 0,001 bis 5 Gew.-% beträgt, bevorzugt 0,01 bis 2 Gew.-%, besonders bevorzugt 0,05 bis 1,5 Gew.-%.

4. Zubereitung nach einem der vorhergehenden Ansprüche in Form von Haarsprays **dadurch gekennzeichnet, dass** das fixierende Polymer ein amphoteres Polymer ist.

5. Zubereitung nach einem der vorhergehenden Ansprüche in Form von Schaumfestigern **dadurch gekennzeichnet, dass** das fixierende Polymer ein nichtionisches Polymer ist.

6. Zubereitung nach einem der vorhergehenden Ansprüche in Form von Frisiergelen **dadurch gekennzeichnet, dass** das fixierende Polymer ein nichtionisches und/oder anionisches Polymer ist.

7. Zubereitung nach einem der vorhergehenden Ansprüche **dadurch gekennzeichnet, dass** das anionische Polymer aus der Gruppe
Vinylacetat/ Crotonsäure Copolymere,
Terpolymere aus Vinylacetat, Crotonat und Vinylalkanoat,
partialveresterte Copolymere zwischen Vinylmethylether und Malein-säureanhydrid, Copolymere aus Acrylsäure oder Methacrylsäure mit Alkylacrylaten und/oder N-Alkylacrylamiden,
Terpolymere aus Acryl-säure, Alkylacrylaten und N-Alkylacrylamiden,
tert.-Butylacrylat/Ethylacrylat/ Methacrylsäure Terpolymer,
Polystyrolsulfonate,
in Wasser lösliche oder dispergierbare anionische Polyurethane
gewählt wird.

8. Zubereitung nach einem der vorhergehenden Ansprüche **dadurch gekennzeichnet, dass** das amphotere Polymer aus der Gruppe
Octylacrylamide/Acrylates/Butylaminoethyl Methacrylate Copolymer,
Acrylates/Lauryl Acrylate/Stearyl Acrylate/Ethylamine Oxide Methacrylate Copolymer, Methacryloyl Ethylbetaine/MethAcrylates Copolymer,
Copolymerisate aus Carboxylgruppen oder Sulfongruppen enthaltenden Monomeren, Copolymere aus N-Octylacrylamid, Methylmethacrylat, Hydroxypropylmethacrylat, N-tert.-Butylaminoethylmethacrylat und Acrylsäure
gewählt wird.

9. Zubereitung nach einem der vorhergehenden Ansprüche **dadurch gekennzeichnet, dass** das nichtionisches Polymer aus der Gruppe
VP/VA Copolymer, PVP, Polyvinylalkohol, Polyvinylcaprolactam, VP/Vinylcaprolactam/Vinyl Imidazole, VP/ Methacrylamide/N-Vinyl Imidazole Copolymer, PVP/Dimethylaminoethylmethacrylate Copolymer gewählt wird.

10. Zubereitung nach einem der vorhergehenden Ansprüche **dadurch gekennzeichnet, dass** ein zusätzliches kationisches Polymer enthalten ist, besonders bevorzugt aus der Gruppe Polyquaternium-4, Polyquaternium-68, Polyquaternium-16, Polyquaternium-11, Polyquaternium-46 gewählt.

11. Zubereitung nach einem der **dadurch gekennzeichnet, dass** die Zubereitung Crotein Kaschmir oder Cashmilan LS 9604 enthält.

12. Verwendung der Zubereitung nach einem der vorangehenden Patentansprüche zur Gestaltung der Haarfrisur.

13. Verwendung von Proteinhydrolysaten aus Kaschmir-Wolle zur Verbesserung der Eigenschaften der fixierenden Polymere in kosmetischen Haarstylingzubereitungen.

14. Verwendung von Proteinhydrolysaten aus Kaschmir-Wolle nach einem der Patentansprüche 2, 3, 7 oder 8 zur Verbesserung der Eigenschaften der fixierenden Polymere in kosmetischen Haarstylingzubereitungen.
